# EUROPÄISCHE PATENTSCHRIFT

(11) **EP 2 358 417 B1**
(45) Veröffentlichungstag und Bekanntmachung des Hinweises auf die Patenterteilung: **03.05.2017**
(21) Anmeldenummer: 09783428.7
(22) Anmeldetag: 25.09.2009
(51) Int. Cl.: A61M 5/42

(54) **MESSSYSTEM, SPRITZENANORDNUNG, MESSSYSTEMANORDNUNG UND VERFAHREN ZUM AUFFINDEN EINER GEEIGNETEN EINSTICHSTELLE EINER NADEL IN EINEN KÖRPER**
MEASUREMENT SYSTEM, INJECTION ARRANGEMENT, MEASUREMENT SYSTEM ARRANGEMENT AND METHOD FOR FINDING A SUITABLE PUNCTURE POINT OF A NEEDLE INTO A BODY
SYSTÈME DE MESURE, ENSEMBLE D'INJECTION, ENSEMBLE SYSTÈME DE MESURE ET PROCÉDÉ POUR DÉTECTER UN POINT DE PIQÛRE ADAPTÉ D'UNE AIGUILLE DANS UN CORPS

(30) Priorität: 26.09.2008 DE 102008049191; 26.01.2009 DE 102009006176
(43) Veröffentlichungstag der Anmeldung: 24.08.2011
(73) Patentinhaber: Ketek Gmbh, 81737 München (DE)
(72) Erfinder: EIDEN, Annette, 82335 Berg (DE)
(74) Vertreter: Epping - Hermann - Fischer
(86) Internationale Anmeldenummer: PCT/EP2009/062453
(87) Internationale Veröffentlichungsnummer: WO 2010/034816

(56) Entgegenhaltungen:
- WO-A1-98/22021
- MESSLINGER K: "Was ist ein Nozizeptor?" DER ANAESTHESIST, Bd. 46, Nr. 2, 1997, Seiten 142-153, XP002566915 ISSN: 0003-2417

## Beschreibung

Der Einstich einer Nadel in die Haut eines Menschen oder Tieres gehört zum Standard jeder ärztlichen Tätigkeit. Dieser Einstich wird vom Körper als ein kurzer Stich empfunden und ist mehr oder weniger schmerzvoll. Gerade in der Pädiatrie wird mit der ersten Impfung eine sehr negative Assoziation zum Arzt geweckt. Aktuell entscheidet das Geschick sowie die Erfahrung des Arztes und auch Glück bei der Wahl der Einstichstelle, ob der Einstich und die Injektion mit einem Schmerzgefühl verbunden sind.

Aus der Druckschrift WO 98/22021 A1 geht eine Injektionsvorrichtung zur Behandlung von Muskelschmerzen hervor. Die Injektionsvorrichtung weist eine Nadel und mehrere an der Nadel angeordnete Elektroden auf, wobei mittels der Elektroden ein aktiver Triggerpunkt im Muskel ermittelt wird.

Es ist eine zu lösende Aufgabe, geeignete Einstichstellen für eine Nadel zu ermitteln, bei denen eine geringere Schmerzempfindung des Patienten auftritt.

Es wird ein Messsystem zum Auffinden einer geeigneten Einstichstelle einer Nadel in einen Körper angegeben, das mindestens eine Messsonde als erste Elektrode und mindestens eine Gegenelektrode aufweist. Zudem umfasst das Messsystem mindestens einen Spannungsdetektor, mit dem das Unterschreiten eines vorgegebenen Grenzwertes der Spannung zwischen der Messsonde und der Gegenelektrode feststellbar ist.

Auf der Hautoberfläche liegen in wechselnder Dichte die Rezeptoren für Druck und Berührung (Mechanorezeptoren), für Wärme und Kälte (Thermorezeptoren) und für Schmerz (Nozirezeptoren). Sticht man mit einer Nadel in die Hautoberfläche ein, so empfindet man einen gut lokalisierbaren Schmerz von "hellem" Charakter, der nach Aufhören des Reizes rasch abklingt. Diesem folgt oft ein zweiter Schmerz von "dumpfem", brennenden Charakter, der schwer zu lokalisieren ist und nur langsam abklingt. Der erste "helle" Schmerz gibt zu Fluchtreaktionen Anlass und führt dazu, dass insbesondere Kinder die Hand bei einer Impfung wegziehen. Die Hautoberfläche ist für den Schmerz nicht gleichmäßig empfindlich, sondern vor allem an den Schmerzpunkten empfindlich. Deshalb sollte eine Injektion an diesen Schmerzpunkten vermieden werden.

Durch die Erfassung der elektrischen Spannung zwischen einem Punkt der Hautoberfläche und einem weiteren Areal der Hautoberfläche, kann festgestellt werden, ob sich in der Nähe dieses Punktes ein Schmerzpunkt befindet. Liegt eine positive Spannung an, beispielsweise 120 mV, so kann davon ausgegangen werden, dass ein Schmerzpunkt vorliegt. Liegt dagegen eine negative Spannung an, ist hier mit hoher Wahrscheinlichkeit eine schmerzfreie oder nur wenig schmerzhafte Injektion möglich.

Zum Auffinden einer geeigneten Einstichstelle wird die Gegenelektrode des Messsystems mit dem Körper elektrisch verbunden. Vorzugsweise wird der elektrische Kontakt mit einem Areal der Hautoberfläche hergestellt, so dass die Spannung über dieses Areal gemittelt wird und unabhängig von der exakten Position der Gegenelektrode ist. Beispielsweise erstreckt sich der elektrische Kontakt der Gegenelektrode mit der Hautoberfläche über eine Fläche von 1 cm². Die Gegenelektrode kann als Kupferfinger ausgebildet sein, den der Patient bei der Messung in der Hand hält.

Zudem wird ein elektrischer Kontakt der Messsonde mit einer Stelle an der Oberfläche des Körpers gebildet, in den potentiell eine Injektion erfolgen kann. Die Messsonde weist vorzugsweise eine Kontaktfläche von weniger als 2 mm² auf.

Mittels des Spannungsdetektors wird festgestellt, ob ein vorgegebener Grenzwert der zwischen der Messsonde und der Gegenelektrode anliegenden Spannung unterschritten wird. Bei Unterschreiten dieses Grenzwertes ist mit hoher Wahrscheinlichkeit eine schmerzreduzierte Injektion an der Stelle möglich, die mit der Kontaktfläche der Messsonde in elektrischem Kontakt steht.

Vorzugsweise erfolgt die Messung der Spannung stimulationsfrei, d. h. ohne vorausgehende oder gleichzeitige elektrische Stimulation der Oberfläche des Körpers. Die elektrisch gemessene, stimulationsfrei anliegende Spannung kann beispielsweise auf vorhandene Aktionspotentiale an Nervenzellen zurückgeführt werden. Zur Ermittlung der anliegenden Spannung ist es ausreichend, die Spannung zu einem einzigen Zeitpunkt zu messen. Insbesondere muss kein zeitlicher Verlauf der Spannung ermittelt werden. Um eine geeignete Einstichstelle mit großer Sicherheit zu ermitteln, kann die Spannung auch über einen kurzen Zeitbereich gemittelt werden.

Vorzugsweise weist das Messsystem eine Anzeige auf, die bei Unterschreiten des Grenzwertes ein Signal abgibt.

Auf diese Weise wird der Arzt oder der Patient darüber informiert, dass eine geeignete Einstichstelle gefunden ist. Beispielsweise wird diese Stelle dann für die Injektion markiert.

Vorzugsweise ist das Signal aus der Menge der optischen und akustischen Signale gewählt.

Beispielsweise leuchtet eine Anzeige auf, wenn sich die Messsonde an einer geeigneten Einstichstelle befindet. Die Anzeige kann direkt an der Messsonde angeordnet sein, aber auch von ihr entfernt sein. Es ist auch ein elektronisches Display als Anzeigegerät möglich. Alternativ oder zusätzlich dazu kann beim Auffinden der Einstichstelle ein Piepton oder ein anderes akustisches Signal ertönen.

Vorzugsweise liegt der Grenzwert im Spannungsbereich von -60 mV bis 0 mV.

Eine negative Spannung deutet darauf hin, dass die Stelle der Körperoberfläche, mit der die Messsonde in elektrischem Kontakt steht, nicht in unmittelbarer Nähe eines Schmerzpunktes liegt. Dabei befindet sich diese Stelle auf einem niedrigeren elektrischen Potential, als das Areal der Hautoberfläche, mit dem die Gegenelektrode einen elektrischen Kontakt bildet. Es hat sich herausgestellt, dass bei einem Unterschreiten von -40 mV bis -60 mV mit hoher Wahrscheinlichkeit eine geeignete Einstichstelle vorliegt. Jedoch deutet auch schon ein negativer Spannungswert, der betragsmäßig geringer ist, auf eine geeignete Einstichstelle hin.

Um das Unterschreiten des Grenzwertes zuverlässig feststellen zu können, weist der Spannungsdetektor vorzugsweise eine Messauflösung von mindestens 30 mV auf und umfasst einen Messbereich von -60 mV bis 30 mV.

Auf diese Weise kann mit großer Sicherheit verhindert werden, dass versehentlich ein Schmerzpunkt, an dem eine geringe positive Spannung anliegt, fälschlicherweise als schmerzunempfindlich angesehen wird.

In einer Ausführungsform ist der Spannungsdetektor ein Elektrometer.

Das Elektrometer ist hochohmig und in der Lage, Spannungen im relevanten Spannungsbereich messen zu können. Das Elektrometer kann auch einen Verstärker, eine Auswerteelektronik und ein Display zur Anzeige der gemessenen Spannung, insbesondere zur Anzeige des Unterschreitens des Grenzwertes, umfassen.

Vorzugsweise ist die Messsonde an einer Haltevorrichtung befestigt.

Mittels der Haltevorrichtung kann die Messsonde einfach auf der Oberfläche des Körpers positioniert werden. Beispielsweise ist die Haltevorrichtung ein Kunststoffteil, in dem Leitungen integriert sein können, die von der Messsonde zum Spannungsdetektor führen.

In einer Ausführungsform ist die Haltevorrichtung an eine Spritze montierbar.

Auf diese Weise kann die Spritze während der Ermittlung einer geeigneten Einstichstelle so mit der Messsonde mitgeführt werden, dass die Injektion direkt nach der Ermittlung des Einstichpunktes erfolgen kann, ohne dass die Position der Spritze verändert werden muss. Dies ermöglicht eine besonders rasche Durchführung der Injektion und eine einfache Handhabung des Messsystems.

Vorzugsweise ist die Haltevorrichtung so ausgebildet, dass sie mit verschiedenen Spritzentypen und Spritzengrößen eingesetzt werden kann.

Die Haltevorrichtung kann ein dehnbares Material aufweisen, das auf das vordere Ende der Spritze, an der die Spritzennadel angeordnet ist, aufgestülpt wird und die Messsonde fest mit der Spritze verbindet. Weiterhin können auch klammerartige Elemente vorgesehen sein, die auf die Spritze aufgeklemmt werden oder Schnappelemente, die beim Aufstecken auf die Spritze in Gegenelemente der Spritze einschnappen. Beispielsweise ist die Haltevorrichtung auf die Spritze aufsteckbar, so dass schon mit einem geringen Zeit- und Kraftaufwand eine sichere Verbindung hergestellt werden kann. Es ist auch eine Schraubverbindung möglich.

In einer Ausführungsform ist die Haltevorrichtung als Hülse ausgebildet.

Die Kontaktfläche der Messsonde befindet sich dabei am vorderen Ende der Hülse und kann sich über die gesamte, beispielsweise ringförmige, vordere Querschnittsfläche der Hülse erstrecken. In diesem Fall kann eine mittlere Spannung zwischen einem kleinen, ringförmigen Bereich der Körperoberfläche und der Gegenelektrode erfasst werden. Beispielsweise weist die Hülse einen Durchmesser von 3 mm auf. Auf diese Weise kann ein geeigneter Einstichbereich ermittelt werden. Die Einstichstelle liegt beispielsweise in einem Bereich innerhalb der Querschnittsfläche der Hülse.

In einer weiteren Ausführungsform ist die Haltevorrichtung als Finger ausgebildet.

Am vorderen Ende des Fingers kann sich eine Kontaktspitze befinden, z. B. mit einem Durchmesser von 1 mm.

Weiterhin wird eine Spritzenanordnung angegeben, die eine Spritze und ein Messsystem umfasst, und bei der die Messsonde mittels der Haltevorrichtung an der Spritze befestigt ist.

Vorzugsweise ist die Haltevorrichtung am vorderen Ende der Spritze, d. h. im Bereich der Spritzennadel, angebracht und ragt in Einstichrichtung über die Spritzennadel hinaus. Die Spritzennadel erstreckt sich entlang einer Längsachse. Vorzugsweise ist der Abstand zwischen der Längsache und der Messsonde gleich oder kleiner als 2 mm.

Auf diese Weise liegt die durch das Messsystem ermittelte, geeignete Einstichstelle so nahe an der tatsächlichen Einstichstelle der Spritzennadel, dass bei unveränderter Position der Spritzenanordnung relativ zur Hautoberfläche eine möglichst schmerzfreie Injektion erfolgen kann. Beispielsweise beträgt der Abstand der ermittelten Einstichstelle von der Längsachse 1 mm.

Bei einer hülsenförmigen Haltevorrichtung ist die Spritzennadel vorzugsweise zumindest teilweise in der Hülsenöffnung angeordnet.

Ist die Haltevorrichtung als Finger ausgeführt, so erstreckt sich dieser vorzugsweise parallel zur Spritzennadel.

Die Messsonde ist relativ zur Spritzennadel verschiebbar gelagert. Auf diese Weise kann ein Bereich einer Hautoberfläche vermessen werden, und, wenn eine geeignete Einstichstelle gefunden ist, die Messsonde relativ zur Spritzennadel verschoben werden, so dass ohne Änderung der Position der Spritze eine Injektion erfolgen kann.

Beispielsweise ist die Messsonde federgelagert an der Spritze befestigt.

Dazu weist die Haltevorrichtung beispielsweise ein Federelement auf, so dass sich durch ein Andrücken der Messsonde an die Hautoberfläche die Messsonde zurückschieben oder zur Seite schieben lässt, so dass eine Injektion ohne Beeinträchtigung durch die Messsonde erfolgen kann. Weiterhin wird eine Messsystemanordnung angegeben, bei der zumindest Teile des Messsystems an einem flexiblen Trägermaterial angebracht sind. Beispielsweise ist das flexible Trägermaterial als Folie ausgebildet, die eine Unterseite und eine Oberseite aufweist. An der Unterseite der Folie sind mehrere Messsonden als erste Elektroden angeordnet.

Die Folie kann mit der Unterseite auf die Haut eines Menschen aufgelegt werden, so dass die Messsonden einen elektrischen Kontakt zur Hautoberfläche bilden. Auf diese Weise kann gleichzeitig zwischen mehreren Punkten der Hautoberfläche und einer Gegenelektrode des Messsystems eine Spannung erfasst und somit eine geeignete Einstichstelle ermittelt werden. Dies hat den Vorteil, dass nicht mehr hintereinander an mehreren Punkten der Hautoberfläche eine Spannung erfasst werden muss.

Vorzugsweise ist die Folie so ausgebildet, dass sie von einer Nadel durchstochen werden kann, so dass nach dem Auffinden einer geeigneten Einstichstelle eine Injektion in die Haut direkt durch die Folie hindurch erfolgen kann.

Die Folie enthält beispielsweise ein flexibles Polymermaterial. Die Folie kann auch als eine Art Pflaster ausgebildet sein, das auf die Hautoberfläche aufgeklebt wird. Dadurch wird ein Verrutschen der Messsonden verhindert.

In einer bevorzugten Ausführungsform ist die Gegenelektrode an der Unterseite der Folie angeordnet.

Eine derartige Messsystemanordnung ist besonders kompakt und einfach zu bedienen. Insbesondere muss kein weiterer elektrischer Kontakt einer separaten Gegenelektrode mit dem Körper ausgebildet werden.

Beispielsweise umgibt die Gegenelektrode die Messsonden rahmenförmig. In weiteren Ausführungsformen kann die Gegenelektrode auch gitterartig ausgebildet sein oder einen anders geformten Teilbereich der Unterseite der Folie belegen. Dabei ist es wichtig, dass die Gegenelektrode eine genügend große Fläche aufweist, so dass die Spannung geeignet gemittelt werden kann.

Vorzugsweise ist jeder Messsonde ein optisches Anzeigeelement zugeordnet, das bei Unterschreiten des Grenzwertes der Spannung zwischen der Messsonde und der Gegenelektrode ein optisches Signal abgibt.

Das Anzeigeelement oder eine dem Anzeigeelement vorgeschaltete Elektronik kann derart ausgebildet sein, dass das Anzeigeelement erst bei Unterschreiten des Grenzwertes der Spannung ein optisches Signal abgibt. Alternativ dazu kann das Anzeigeelement auch schon bei einer Spannung, die über dem Grenzwert liegt, ein optisches Signal abgeben, dass dann allerdings eine verminderte Leuchtstärke aufweist.

Vorzugsweise sind die optischen Anzeigeelemente an der Oberseite der Folie angeordnet.

Beispielsweise ist wenigstens ein optisches Anzeigeelement eine Leuchtdiode.

Vorzugsweise weist die Folie eine Unterteilung in Rasterzellen auf, wobei sich in einer Rasterzelle jeweils ein optisches Anzeigeelement und eine Messsonde befinden.

Beispielsweise ist die Fläche der Folie in 4 x 4 oder 5 x 5 Rasterzellen unterteilt, die als regelmäßiges Gitter angeordnet sind. Die Unterteilung der Folie in Rasterzellen kann von außen sichtbar sein. Dazu kann die Folie Markierungen wie z. B. Einkerbungen oder Farbmarkierungen aufweisen, die die einzelnen Rasterzellen voneinander abgrenzen. Die Unterteilung der Folie kann auch nur eine gedachte Unterteilung sein. Wesentlich dabei ist, dass sich bezüglich der Folienebene das jeweilige optische Anzeigeelement so nahe an der zugeordneten Messsonde befindet, dass der Benutzer die erhaltene Anzeige des optischen Anzeigeelements direkt dem vermessenen Hautbereich zuordnen kann.

Vorzugsweise zeigt das optische Anzeigeelement das Unterschreiten des Grenzwertes der Spannung zwischen der in derselben Rasterzelle angeordneten Messsonde und der Gegenelektrode an.

Somit zeigt das optische Anzeigeelement an, ob in der zugehörigen Rasterzelle der Grenzwert der Spannung unterschritten wird und somit eine geeignete Einstichstelle in dieser Rasterzelle vorliegt. Dabei kann es auch möglich sein, dass gleichzeitig in verschiedenen Rasterzellen optische Anzeigeelemente das Unterschreiten des Grenzwertes der Spannung anzeigen. In diesem Fall liegen mehrere mögliche Einstichstellen vor.

In einer Ausführungsform weist die Messsystemanordnung wenigstens einen Messverstärker auf, der die zwischen einer Messsonde und der Gegenelektrode anliegende Spannung verstärkt.

Dabei kann wenigstens ein Messverstärker in der Folie integriert sein. Beispielsweise kann sich zwischen jeder Messsonde und der Gegenelektrode ein in der Folie integrierter Messverstärker befinden, der jeweils die hochohmige Signalspannung zwischen der Messsonde und der Gegenelektrode verstärkt. Es kann jedoch auch vorgesehen sein, dass der Messverstärker nicht in der Folie integriert ist, sondern mittels eines Kabels extern mit der Folie verbunden ist.

Weiterhin wird ein Verfahren zum Auffinden einer geeigneten Einstichstelle einer Nadel in einen Körper angegeben. Das Verfahren umfasst die Schritte:
A) Bereitstellen eines Messsystems,
B) Bilden eines elektrischen Kontakts der Gegenelektrode mit dem Körper,
C) Bilden eines elektrischen Kontakts der Messsonde mit einer Stelle an der Oberfläche des Körpers und,
D) Ermitteln einer Stelle der Oberfläche, an dem die Spannung zwischen der Messsonde und der Gegenelektrode einen folgenden Grenzwert unterschreitet.

In einer Ausführungsform des Verfahrens wird in Schritt C) nacheinander an verschiedenen Stellen an der Oberfläche des Körpers ein elektrischer Kontakt mit der Messsonde hergestellt.

Somit wird ein Areal der Hautoberfläche auf geeignete Einstichstellen hin untersucht. Sobald eine geeignete Einstichstelle gefunden ist, kann diese Stelle markiert werden und nachfolgend die Injektion mit einer Spritze an dieser Stelle erfolgen.

Die Messsonde kann vor Schritt C) auch an der Spritze befestigt werden, so dass eine Spritzenanordnung gebildet wird. In diesem Fall kann auch ohne Markieren des Punktes die Injektion im Bereich der ermittelten geeigneten Einstichstelle vorgenommen werden. Dabei kann unmittelbar nach dem Ermitteln einer geeigneten Einstichstelle, bei unveränderter Position der Spritze die Injektion vorgenommen werden.

In einer weiteren Ausführungsform des Verfahrens liegt das Messsystem in einer Messsystemanordnung mit einer Folie vor, bei der mehrere Messsonden an der Unterseite der Folie angeordnet sind. Die Folie wird mit ihrer Unterseite auf die Oberfläche des Körpers aufgelegt, so dass gleichzeitig mit mehreren Messsonden ein elektrischer Kontakt zur Oberfläche des Körpers hergestellt wird. Falls die Gegenelektrode ebenfalls an der Unterseite der Folie angeordnet ist, wird dabei auch gleichzeitig ein elektrischer Kontakt der Gegenelektrode mit der Oberfläche des Körpers hergestellt.
Im Fall, dass die optischen Anzeigeelemente an der Oberseite der Folie in Rasterzellen angeordnet sind, wird dem Benutzer durch das Aufleuchten eines Anzeigeelementes angezeigt, dass eine Rasterzelle eine geeignete Einstichstelle darstellt. Vorzugsweise ist die Folie derart ausgebildet, dass sie während der Vornahme einer Injektion an der ermittelten Einstichstelle auf der Oberfläche des Körpers verbleiben kann. Vorzugsweise kann die Folie von der Injektionsnadel durchstochen werden.

Im Folgenden werden das angegebene Messsystem, die angegebene Spritzenanordnung, die angegebene Messsystemanordnung sowie die angegebenen Verfahren und ihre vorteilhaften Ausgestaltungen anhand von schematischen und nicht maßstabsgetreuen Figuren erläutert. Es zeigen:
- Figur 1: ein Blockdiagramm eines Messsystems,
- Figur 2: eine seitliche Aufsicht auf einen Teil einer Spritzenanordnung,
- Figur 3: eine seitliche Aufsicht auf eine weitere Ausführungsform eines Teils einer Spritzenanordnung,
- Figur 4A: eine Aufsicht auf die Unterseite einer Folie, an der Messsonden angeordnet sind,
- Figur 4B: eine Aufsicht auf die Oberseite einer Folie gemäß Figur 4A,
- Figur 4C: einen Querschnitt eines Teils der Folie gemäß Figur 4A.

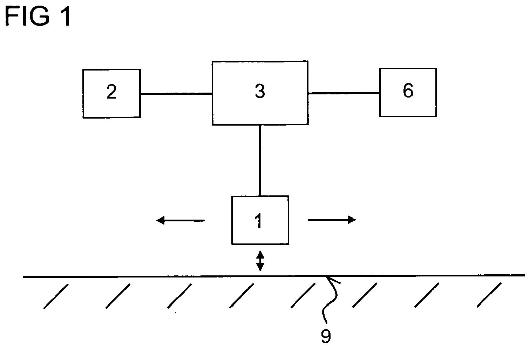

Figur 1 zeigt ein Messsystem und ein Verfahren zum Auffinden einer geeigneten Einstichstelle einer Nadel in einem Körper. Das Messsystem umfasst eine Messsonde 1 als erste Elektrode, eine Gegenelektrode 2, einen Spannungsdetektor 3 und eine Anzeige 6. Zum Auffinden einer geeigneten Einstichstelle wird die Gegenelektrode 2 mit einem Areal der Körperoberfläche in elektrischen Kontakt gebracht. Die Gegenelektrode 2 ist beispielsweise ein Kupferfinger, den ein Patient in die Hand nimmt. Anschließend wird die Messsonde 1 mit einer Stelle an der Hautoberfläche 9 in elektrischen Kontakt gebracht. Mittels eines Spannungsdetektors 3 wird festgestellt, ob ein vorgegebener Grenzwert der zwischen der Messsonde 1 und der Gegenelektrode 2 anliegenden Spannung unterschritten wird. Die Grenzspannung liegt z. B. bei -40 mV. Wird diese Grenzspannung nicht unterschritten, so wird die Messsonde 1 mit einer anderen Stelle der Hautoberfläche 9 in elektrischen Kontakt gebracht und es wird wieder mittels des Spannungsdetektors 3 geprüft, ob die Grenzspannung unterschritten ist. Das Unterschreiten der Grenzspannung wird dem Arzt mittels einer Anzeige 6, beispielsweise durch ein Leuchtsignal mitgeteilt. Anschließend kann eine so ermittelte, geeignete Einstichstelle markiert werden, sodass nach Entfernen der Messsonde 1 von der Hautoberfläche 9 dort eine Injektion vorgenommen werden kann. Allerdings kann die Messsonde 1 auch an einer Spritze angeordnet sein, sodass die Spritze mit der Messsonde mitgeführt wird und sich dann automatisch in der unmittelbaren Umgebung der ermittelten, geeigneten Einstichstelle befindet. Bei einer derartigen Spritzenanordnung kann die Injektion bei unveränderter Position der Spritze unmittelbar nach Auffinden einer geeigneten Einstichstelle erfolgen. In diesem Fall ist keine Markierung der Einstichstelle nötig.

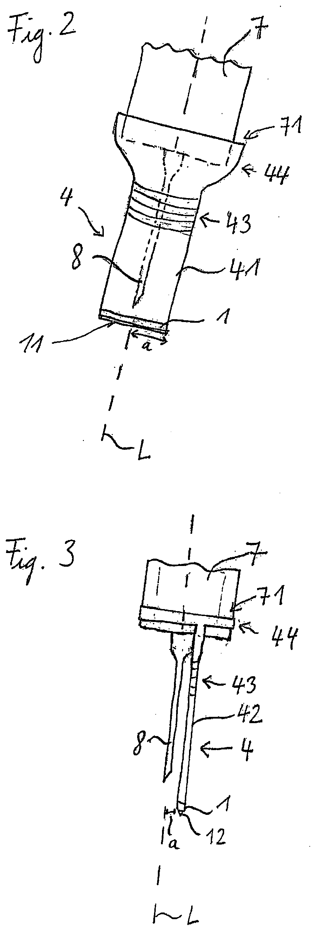

Figur 2 zeigt einen Teil einer Spritzenanordnung. Die Messsonde 1 ist mittels einer Haltevorrichtung 4 an einer Spritze 7 befestigt. In die Haltevorrichtung 4 sind Leitungen integriert (nicht gezeigt), die einen elektrischen Kontakt zwischen der Messsonde 1 und einem Spannungsdetektor (hier nicht gezeigt) herstellen. Die Spritze 7 weist eine Spritzennadel 8 auf, die sich entlang einer Längsachse L erstreckt. Die Längsachse L entspricht der Einstichrichtung der Spritzennadel 8 in die Hautoberfläche. Die Haltevorrichtung 4 ist als Hülse 41 ausgebildet, an deren vorderem Ende die Messsonde 1 angeordnet ist. Die Messsonde 1 weist eine Kontaktfläche 11 auf, die sich über die ringförmige Querschnittsfläche der Hülse 41 erstreckt.

Die Spritzennadel 8 befindet sich in der Hülsenöffnung und wird von der Hülse 41 umgeben. Die Hülse 41 weist einen Radius a = 1,5 mm auf. Der Abstand zwischen der Längsachse L und der Kontaktfläche 11 beträgt somit ebenfalls 1,5 mm.

Die Haltevorrichtung 4 weist einen Befestigungsbereich 44 auf, an dem sie mit der Spritze 7 verbunden ist. Der Befestigungsbereich 44 ist dehnbar und wird zur Befestigung über das vordere Ende 71 der Spritze 7 gestülpt. Die Messsonde 1 ist federgelagert mit der Spritze 7 verbunden. Dazu weist die Haltevorrichtung 4 einen Federbereich 43 auf, der zwischen der Messsonde 1 und dem Befestigungsbereich 44 angeordnet ist.

Zum Auffinden einer geeigneten Einstichstelle wird die Kontaktfläche 11 mit der Hautoberfläche eines Patienten in Berührung gebracht und es wird mittels eines Spannungsdetektors geprüft, ob ein vorgegebener Grenzwert unterschritten ist. Bei Unterschreiten des Grenzwertes ist eine geeignete Einstichstelle gefunden. Die Injektion kann daraufhin ohne Veränderung der Position der Spritzenanordnung vorgenommen werden. Dazu übt der Arzt in Richtung der Längsachse L einen Druck auf die Spritze 7 aus, wodurch sich die federgelagerte Messsonde 1 relativ zur Spritzennadel 8 entgegen der Einstichrichtung zurückschiebt. Die Spritzennadel 8 kommt schließlich mit der Hautoberfläche in Berührung, so dass eine Injektion erfolgen kann. Der tatsächliche Einstichpunkt ist von der ermittelten geeigneten Einstichstelle um den Abstand a zwischen der Längsachse L und der Kontaktfläche 11 entfernt. Aufgrund des geringen Abstandes a ist die Wahrscheinlichkeit dafür hoch, dass die Injektion nicht oder nur wenig schmerzhaft verläuft.

Figur 3 zeigt ein weiteres Ausführungsbeispiel einer Spritzenanordnung, bei der die Haltevorrichtung 4 als Finger 42 ausgebildet ist, der sich parallel zur Längsachse L der Spritzennadel 8 erstreckt. Die Messsonde 1 befindet sich am vorderen Ende des Fingers 42 und weist eine Kontaktspitze 12 auf, die mit der Hautoberfläche in elektrischen Kontakt gebracht wird. Die Kontaktspitze 12 hat eine Querschnittsfläche von ca. 1 mm². Der Finger 42 ist in einem Abstand a = 1 mm von der Spritzennadel 8 angeordnet. Auf diese Weise wird auch hier sichergestellt, dass die tatsächliche Einstichstelle möglichst nahe an der ermittelten, geeigneten Einstichstelle liegt.

Die Haltevorrichtung 4 weist einen Federbereich 43 auf, sodass sich beim Andrücken der Spritze 7 an die Hautoberfläche die Messsonde 1 entgegen der Einstichrichtung zurückschiebt und den Weg für die Spritzennadel 8 freigibt. Somit kann die Injektion unmittelbar nach dem Auffinden einer geeigneten Einstichstelle erfolgen.

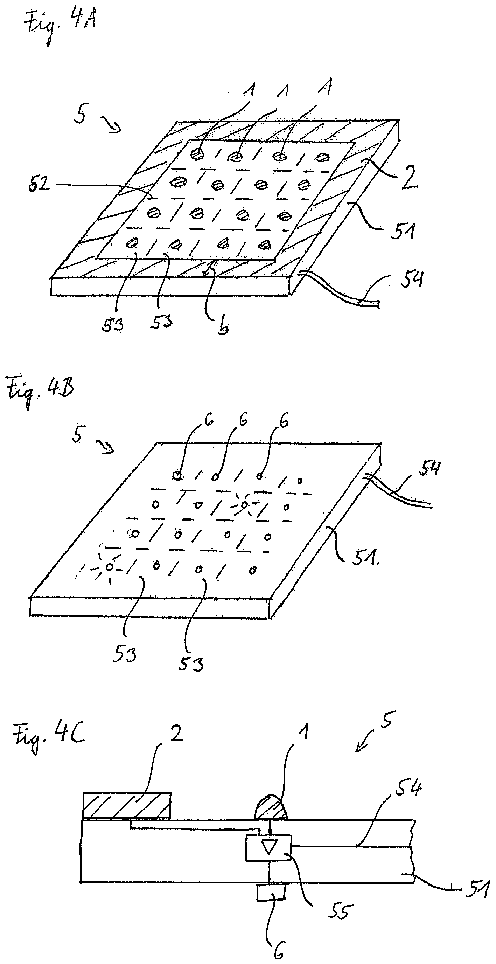

Die Figuren 4A bis 4C zeigen aus verschiedenen Ansichten eine Messsystemanordnung 5 mit einer Folie 51.

Figur 4A zeigt eine Aufsicht auf die Unterseite der Folie 51, an der mehrere Messsonden 1 angeordnet sind. Die Messsonden 1 sind als Goldbumps mit einem Durchmesser von 20 µm ausgebildet und werden mittels Zuleitungen (in dieser Figur nicht gezeigt) elektrisch kontaktiert. Die Folie 51 weist eine Fläche von 2 x 2 cm² auf und ist in ein Raster 52 mit Rasterzellen 53 unterteilt. Das Raster 52 ist als regelmäßges Gitter mit 4 x 4 Rasterzellen 53 ausgebildet, in denen sich jeweils eine Messsonde 1 befindet. Die Unterteilung in Rasterzellen 53 ist an der Unterseite für den Benutzer nicht sichtbar. Das Raster 52 ist von einer rahmenförmigen Gegenelektrode 2 mit einer Rahmenbreite b von ca. 5 mm umgeben. Zur Ermittlung einer geeigneten Einstichstelle für eine Injektion wird die Folie 51 mit ihrer Unterseite auf die Hautoberfläche aufgelegt, so dass die Gegenelektrode 2 und die einzelnen Messsonden 1 in elektrischem Kontakt mit der Hautoberfläche stehen.

Figur 4B zeigt eine Aufsicht auf die Oberseite der Folie 51. Die Oberseite weist ebenfalls die Unterteilung in Rasterzellen 53 auf, die durch Einkerbungen der Folie 51 für den Benutzer sichtbar ist. In jeder Rasterzelle 53 befindet sich ein optisches Anzeigeelement 6 in Form einer Leuchtdiode. Die optischen Anzeigeelemente 6 werden mittels der Spannung angesteuert, die zwischen der in derselben Rasterzelle 53 angeordneten Messsonde 1 und der Gegenelektrode 2 anliegt. Wenn diese Spannung einen vorgegebenen Grenzwert unterschreitet, leuchtet das optische Anzeigeelement 6 auf und zeigt damit an, dass der Hautbereich unter der zugehörigen Rasterzelle 53 eine geeignete Einstichstelle für eine Injektion darstellt. Wenn zwischen mehreren Messsonden 1 und der Gegenelektrode 2 die Spannung unter dem Grenzwert liegt, können auch mehrere optische Anzeigeelemente 6 gleichzeitig aufleuchten. Durch die Leuchtstärke kann dem Benutzer angezeigt werden, wie stark der Grenzwert unterschritten wird, so dass eine Injektion vorzugsweise in der Rasterzelle 53 vorgenommen wird, bei der das optische Anzeigeelement 6 am stärksten aufleuchtet.

Figur 4C zeigt im Querschnitt einen Abschnitt der Folie 51, in dem eine Messsonde 1 und ein Teil der Gegenelektrode 2 zu sehen ist. Die zwischen der Gegenelektrode 2 und einer Messsonde 1 anliegende Spannung wird mittels eines Messverstärkers 55 verstärkt und zum optischen Anzeigeelement 6 geführt. Der Messverstärker 55 wird mittels einer Leitung 54, die durch die Folie 51 hindurch geführt wird, von außen mit Spannung versorgt. Der Messverstärker 55 ist so ausgelegt, dass die zum Betrieb des optischen Anzeigeelementes 6 benötigte Spannung erst bei einem Unterschreiten der zwischen der Messsonde 1 und der Gegenelektrode 2 anliegenden Signalspannung unter einen festgelegten Grenzwert geliefert wird. Die weiteren Messsonden 1 und optischen Anzeigeelemente 6 sind hier der Übersichtlichkeit halber nicht dargestellt. Sie sind auf gleiche Weise mit der Gegenelektrode 2, einem Messverstärker 55 und der Leitung 54 zur Spannungsversorgung verbunden.
Die Erfindung ist nicht durch die Beschreibung an Hand der Ausführungsbeispiele auf diese beschränkt, sondern umfasst jedes neue Merkmal sowie jede Kombination von Merkmalen gemäß den Ansprüchen.

### Bezugszeichenliste

- 1: Messsonde
- 11: Kontaktfläche
- 12: Kontaktspitze
- 2: Gegenelektrode
- 3: Spannungsdetektor
- 4: Haltevorrichtung
- 41: Hülse
- 42: Finger
- 43: Federbereich
- 44: Befestigungsbereich
- 5: Messsystemanordnung
- 51: Folie
- 52: Raster
- 53: Rasterzelle
- 54: Leitung zur Spannungsversorgung
- 55: Messverstärker
- 6: Anzeige
- 61: Anzeigeelemente
- 7: Spritze
- 71: vorderes Ende der Spritze
- 8: Spritzennadel
- 9: Hautoberfläche

- L: Längsachse
- a: Abstand zwischen Längsachse und Messsonde
- b: Rahmenbreite der Gegenelektrode

## Patentansprüche

1. Spritzenanordnung
- umfassend eine Spritze (7) aufweisend eine Spritzennadel (8) und ein Messsystem zum Auffinden einer geeigneten Einstichstelle einer Nadel in einen Körper mit
- mindestens einer Messsonde (1) als erste Elektrode,
- mit mindestens einer Gegenelektrode (2) und
- mit mindestens einem Spannungsdetektor (3), mit dem das Unterschreiten eines vorgegebenen Grenzwertes der zwischen der Messsonde (1) und der Gegenelektrode (2) anliegenden Spannung feststellbar ist, und
- umfassend eine Haltevorrichtung (4), wobei die Messsonde (1) mittels der Haltevorrichtung (4) an der Spritze (7) befestigt ist,
- wobei die Messsonde (1) relativ zur Spritzennadel (8) verschiebbar gelagert ist.

2. Spritzenanordnung nach Anspruch 1,
- bei der das Messsystem eine Anzeige (6) aufweist, die bei Unterschreiten des Grenzwertes ein optisches oder akustisches Signal abgibt.

3. Spritzenanordnung nach einem der Ansprüche 1 bis 2,
- bei der der Grenzwert im Spannungsbereich von -60 mV bis 0 mV liegt.

4. Spritzenanordnung nach einem der Ansprüche 1 bis 3,
- bei der der Spannungsdetektor (3) eine Messauflösung von mindestens 30 mV aufweist und einen Messbereich von -60 mV bis +30 mV umfasst.

5. Spritzenanordnung nach einem der Ansprüche 1 bis 3 ,
- bei der der Spannungsdetektor (3) ein Elektrometer ist, das eine Messauflösung von mindestens 30 mV aufweist und einen Messbereich von -60 mV bis +30 mV umfasst.

6. Spritzenanordnung nach einem der Ansprüche 1 bis 5,
- bei der die Haltevorrichtung (4) als Hülse (41) mit einer Hülsenöffnung ausgebildet ist und bei der die Spritzennadel (8) zumindest teilweise in der Hülsenöffnung angeordnet ist.

7. Spritzenanordnung nach Anspruch 6,
- bei der die Messsonde (1) federgelagert an der Spritze (7) befestigt ist.

8. Messsystemanordnung
- umfassend eine Folie (51) aufweisend eine Unterseite und eine Oberseite und
- mit einem Messsystem zum Auffinden einer geeigneten Einstichstelle einer Nadel in einen Körper mit
- mindestens einer Messsonde (1) als erste Elektrode,
- mit mindestens einer Gegenelektrode (2) und
- mit mindestens einem Spannungsdetektor (3), mit dem das Unterschreiten eines vorgegebenen Grenzwertes der zwischen der Messsonde (1) und der Gegenelektrode (2) anliegenden Spannung feststellbar ist,
- bei der das Messsystem mehrere Messsonden (1) als erste Elektroden aufweist,
- bei der die Messsonden (1) an der Unterseite der Folie (51) angeordnet sind.

9. Messsystemanordnung nach Anspruch 8,
- bei der die Folie (51) eine Unterteilung in Rasterzellen (53) aufweist und
- bei der sich in einer Rasterzelle (53) jeweils ein optisches Anzeigeelement (61) und eine Messsonde (1) befindet.

10. Messsystemanordnung nach einem der Ansprüche 8 oder 9,
- bei der in der Folie (51) wenigstes ein Messverstärker (55) integriert ist, der die zwischen einer Messsonde (1) und der Gegenelektrode (2) anliegende Spannung verstärkt.

11. Messsystemanordnung nach einem der Ansprüche 8 bis 10,
- bei der die Folie (51) derart ausgebildet ist, dass sie von einer Nadel durchstochen werden kann.

12. Verfahren zum Auffinden einer geeigneten Einstichstelle einer Nadel in einen Körper, umfassend die Schritte
A) Bereitstellen eines Messsystem zum Auffinden einer geeigneten Einstichstelle einer Nadel in einen Körper mit
- mindestens einer Messsonde (1) als erste Elektrode,
- mit mindestens einer Gegenelektrode (2) und
- mit mindestens einem Spannungsdetektor (3), mit dem das Unterschreiten eines vorgegebenen Grenzwertes der zwischen der Messsonde (1) und der Gegenelektrode (2) anliegenden Spannung feststellbar ist,
B) Bilden eines elektrischen Kontakts der Gegenelektrode (2) mit der Körperoberfläche,
C) Bilden eines elektrischen Kontakts der Messsonde (1) mit einer Stelle an der Oberfläche des Körpers und
D) Ermitteln einer Stelle der Oberfläche, an der die Spannung zwischen der Messsonde (1) und der Gegenelektrode (2) einen vorgegebenen Grenzwert unterschreitet.

13. Verfahren nach Anspruch 12,
- wobei in Schritt C) nacheinander an verschiedenen Punkten an der Oberfläche des Körpers ein elektrischer Kontakt mit der Messsonde (1) hergestellt wird.

14. Verfahren nach einem der Ansprüche 12 oder 13,
- wobei vor Schritt C) eine Spritzenanordnung nach einem der Ansprüche 1 bis 7 gebildet wird.

15. Verfahren nach Anspruch 12,
- bei dem das Messsystem als Messsystemanordnung (5) nach einem der Ansprüche 8 bis 11 vorliegt und
- wobei in Schritt C) die Folie (51) mit ihrer Unterseite auf die Oberfläche des Körpers aufgelegt wird, so dass gleichzeitig mit mehreren Messsonden (1) ein elektrischer Kontakt zur Oberfläche des Körpers hergestellt wird.

## Claims

1. A syringe arrangement
- comprising a syringe (7) having a syringe needle (8) and a measuring system for finding a suitable puncture point for a needle into a body, with
- at least one measurement probe (1) as a first electrode,
- with at least one counter-electrode (2) and
- with at least one voltage detector (3) with which it can be determined whether the voltage in effect between the measurement probe (1) and the counter-electrode (2) is below a pre-defined limit, and
- comprising a clamping device (4), wherein the measurement probe (1) is attached to the syringe (7) by means of the clamping device (4),
- wherein the measurement probe (1) is slideably mounted relative to the syringe needle (8).

2. The syringe arrangement according to claim 1,
- in which the measuring system has a display (6), which emits a visual or acoustic signal when the measured value is below the limit.

3. The syringe arrangement according to one of claims 1 to 2,
- in which the limit lies within the voltage range of -60 mV to 0 mV.

4. The syringe arrangement according to one of claims 1 to 3,
- in which the voltage detector (3) has a measurement resolution of at least 30 mV and comprises a measurement range of -60 mV to +30 mV.

5. The syringe arrangement according to one of claims 1 to 3,
- in which the voltage detector (3) is an electrometer which has a measurement resolution of at least 30 mV and comprises a measurement range of -60 mV to +30 mV.

6. The syringe arrangement according to one of claims 1 to 5,
- in which the clamping device (4) is designed as a sleeve (41) with a sleeve opening and in which the syringe needle (8) is arranged at least partially in the sleeve opening.

7. The syringe arrangement according to claim 6,
- in which the measurement probe (1) is attached to the syringe in a spring-loaded manner.

8. A measuring system arrangement
- comprising a film (51) having a lower side and an upper side and
- with a measuring system for finding a suitable puncture point for a needle into a body, with
- at least one measurement probe (1) as a first electrode,
- with at least one counter-electrode (2) and
- with at least one voltage detector (3) with which it can be determined whether the voltage in effect between the measurement probe (1) and the counter-electrode (2) is below a pre-defined limit,
- in which the measuring system has multiple measurement probes (1) as first electrodes,
- in which the measurement probes (1) are arranged on the lower side of the film (51).

9. The measuring system arrangement according to claim 8,
- in which the film (51) is subdivided into grid cells (53) and
- in which an optical display element (61) and a measurement probe (1) are located in each grid cell (53).

10. The measuring system arrangement according to one of claims 8 or 9,
- in which in the film (51) at least one measuring amplifier (55) is integrated, which amplifies the voltage in effect between a measurement probe (1) and the counter-electrode (2).

11. The measuring system arrangement according to one of claims 8 to 10,
- in which the film (51) is designed such that it can be punctured by a needle.

12. A method for finding a suitable puncture point for a needle into a body, comprising the steps of
A) providing a measuring system for finding a suitable puncture point for a needle into a body with
- at least one measurement probe (1) as a first electrode,
- with at least one counter-electrode (2) and
- with at least one voltage detector (3) with which it can be determined whether the voltage in effect between the measurement probe (1) and the counter-electrode (2) is below a pre-defined limit,
B) forming an electrical contact of the counter-electrode (2) with the body surface,
C) forming an electrical contact of the measurement probe (1) with a point on the surface of the body and
D) determining a point on the surface at which the voltage between the measurement probe (1) and the counter-electrode (2) is below a predefined limit.

13. The method according to claim 12,
- wherein in step C) an electrical contact is made with the measurement probe (1) at various points in succession on the surface of the body.

14. The method according to one of claims 12 or 13,
- wherein before step C) a syringe arrangement according to one of claims 1 to 7 is formed.

15. The method according to claim 12,
- in which the measuring system is present as the measuring system arrangement (5) according to one of claims 8 to 11 and
- wherein in step C) the film (51) is placed with its lower side on the surface of the body, so that an electrical contact with the surface of the body is made with multiple measurement probes (1) simultaneously.

## Revendications

1. Agencement de seringue
- comprenant une seringue (7) présentant une aiguille de seringue (8) et un système de mesure destiné à trouver un point de piqûre approprié d'une aiguille dans un corps, comprenant
- au moins une sonde de mesure (1) en tant que première électrode,
- comprenant au moins une contre-électrode (2) et
- comprenant au moins un détecteur de tension (3) à l'aide duquel le dépassement vers le bas d'une valeur limite prédéfinie de la tension présente entre la sonde de mesure (1) et la contre-électrode (2) peut être constatée, et
- comprenant un dispositif de maintien (4), la sonde de mesure (1) étant fixée sur la seringue (7) au moyen du dispositif de maintien (4),
- la sonde de mesure (1) étant logée de manière coulissante par rapport à l'aguille de seringue (8).

2. Agencement de seringue selon la revendication 1,
- dans lequel le système de mesure présente un indicateur (6) qui sort un signal optique ou acoustique lors du dépassement vers le bas de la valeur limite.

3. Agencement de seringue selon l'une quelconque des revendications 1 à 2,
- dans lequel la valeur limite est située dans la plage de tension de -60 mV à 0 mV.

4. Agencement de seringue selon l'une quelconque des revendications 1 à 3,
- dans lequel le détecteur de tension (3) présente une résolution de mesure d'au moins 30 mV et comprend une plage de mesure de -60 mV à +30 mV.

5. Agencement de seringue selon l'une quelconque des revendications 1 à 3,
- dans lequel le détecteur de tension (3) est un électromètre qui présente une résolution de mesure d'au moins 30 mV et comprend une plage de mesure de -60 mV à +30 mV.

6. Agencement de seringue selon l'une quelconque des revendications 1 à 5,
- dans lequel le dispositif de maintien (4) est réalisé comme douille (41) munie d'une ouverture de douille et dans lequel l'aiguille de seringue (8) est disposée au moins en partie dans l'ouverture de douille.

7. Agencement de seringue selon la revendication 6,
- dans lequel la sonde de mesure (1) est fixée sur la seringue (7) en étant logée par ressort.

8. Agencement de système de mesure
- comprenant un film (51) présentant une face inférieure et une face supérieure et
- comprenant un système de mesure destiné à trouver un point de piqûre approprié d'une aiguille dans un corps, comprenant
- au moins une sonde de mesure (1) en tant que première électrode,
- comprenant au moins une contre-électrode (2) et
- comprenant au moins un détecteur de tension (3) à l'aide duquel le dépassement vers le bas d'une valeur limite prédéfinie de la tension présente entre la sonde de mesure (1) et la contre-électrode (2) peut être constatée,
- dans lequel le système de mesure présente plusieurs sondes de mesure (1) en tant que premières électrodes,
- dans lequel les sondes de mesure (1) sont disposées sur la face inférieure du film (51).

9. Agencement de système de mesure selon la revendication 8,
- dans lequel le film (51 présente une sous-division en cellules de grille (53) et
- dans lequel respectivement un élément d'indication (61) optique et une sonde de mesure (1) se trouvent dans une cellule de grille (53).

10. Agencement de système de mesure selon la revendication 8 ou 9,
- dans lequel au moins un amplificateur de mesure (55) est intégré dans le film (51), lequel amplificateur de mesure amplifie la tension présente entre une sonde de mesure (1) et la contre-électrode (2).

11. Agencement de système de mesure selon l'une quelconque des revendications 8 à 10,
- dans lequel le film (51) est réalisé de manière à ce qu'il puisse être percé par une aiguille.

12. Procédé destiné à trouver un point de piqûre approprié d'une aiguille dans un corps, comprenant les étapes
A) mise à disposition d'un système de mesure destiné à trouver un point de piqûre approprié d'une aiguille dans un corps, comprenant
- au moins une sonde de mesure (1) en tant que première électrode,
- comprenant au moins une contre-électrode (2) et
- comprenant au moins un détecteur de tension (3) à l'aide duquel le dépassement vers le bas d'une valeur limite prédéfinie de la tension présente entre la sonde de mesure (1) et la contre-électrode (2) peut être constaté
B) formation d'un contact électrique de la contre-électrode (2) avec la surface du corps,
C) formation d'un contact électrique de la sonde de mesure (1) avec un point à la surface du corps et
D) détermination d'un point de la surface, sur lequel la tension entre la sonde de mesure (1) et la contre-électrode (2) dépasse vers le bas une valeur limite prédéfinie.

13. Procédé selon la revendication 12,
- un contact électrique étant établi à l'aide de la sonde de mesure, à l'étape C), successivement à différents points à la surface du corps.

14. Procédé selon l'une quelconque des revendications 12 ou 13,
- un agencement de seringue selon l'une quelconque des revendications 1 à 7 étant formé avant l'étape C).

15. Procédé selon la revendication 12,
- dans lequel le système de mesure est présent en tant qu'agencement de système de mesure (5) selon l'une quelconque des revendications 8 à 11 et
- le film (51), à l'étape C), étant posé avec sa face inférieure sur la surface du corps, de sorte qu'un contact électrique vers la surface du corps est établi simultanément à l'aide de plusieurs sondes de mesure (1).
